(19) Europäisches Patentamt
European Patent Office
Office européen des brevets

(11) Veröffentlichungsnummer: **0 416 261 A1**

# (12) EUROPÄISCHE PATENTANMELDUNG

(21) Anmeldenummer: **90114123.4**

(22) Anmeldetag: **24.07.90**

(51) Int. Cl.[5]: **C07C 41/56**, C07C 43/303

(30) Priorität: **29.07.89 DE 3925207**

(43) Veröffentlichungstag der Anmeldung:
**13.03.91 Patentblatt 91/11**

(84) Benannte Vertragsstaaten:
**AT BE DE FR GB IT NL SE**

(71) Anmelder: **HOECHST AKTIENGESELLSCHAFT**
**Postfach 80 03 20**
**W-6230 Frankfurt am Main 80(DE)**

(72) Erfinder: **Lachhein, Stephen, Dr.**
**Kiedricher Strasse 18**
**W-6238 Hofheim am Taunus(DE)**

## (54) Verfahren zur Herstellung von Ketalen.

(57) Die Erfindung betrifft ein Ketalisierungsverfahren für insbesondere sterisch gehinderte Ketone, wobei ein Keton der Formel $R^1COCH_3$, worin $R^1$ gegebenenfalls ungesättigtes Alkyl bedeutet, mit katalytischen Mengen Alkohol $R^2OH$, worin $R^2$ Alkyl oder Halogenalkyl bedeutet, und mindestens äquimolaren Mengen an Orthoameisensäureester $HC(OR^2)_3$ unter sauren Bedingungen und Abdestillieren des entstehenden Ameisensäureesters $HCOOR^2$ zu Ketalen der Formel $R^1C(CH_3)(OR^2)_2$ umgesetzt wird.

EP 0 416 261 A1

Xerox Copy Centre

## VERFAHREN ZUR HERSTELLUNG VON KETALEN

Ketale der Formel (I)

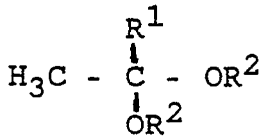

(I)

worin
$R^1$ Wasserstoff, Alkyl, Alkenyl oder Alkinyl und
$R^2$ Alkyl
bedeuten, sind wertvolle Zwischenprodukte bei der Herstellung von Substanzen, welche die phytotoxischen Nebenwirkungen von Pflanzenschutzmitteln wirksam reduzieren (vgl. beispielsweise Deutsche Patentanmeldung P 3923649.8). Die Verbindungen der Formel (I) sind bekannt oder lassen sich nach laborüblichen Methoden herstellen.

So ist bereits bekannt, daß Acetale und Ketale durch Umsetzung der Aldehyde und Ketone beispielsweise mit Alkoholen unter saurer Katalyse dargestellt werden können (siehe Houben-Weyl, Band VI/3, 199 ff; C. Ferri, Reaktionen der organischen Synthese, 426-427, Georg Thieme Verlag Stuttgart 1978).

Bekanntermaßen reagieren jedoch Ketone nur sehr schlecht oder überhaupt nicht nach dieser Variante (siehe Houben-Weyl, Band VI/3, 204, Houben-Weyl, Band VII/1, 419).

Allgemein anwendbare Methoden, die auch die Darstellung von Ketalen einschließen, sind die Umsetzungen in Gegenwart von wasserentziehenden Mitteln, wie z.B. Acetondimethylacetal oder Orthoameisensäureestern (Claisen-Verfahren), unter sauer-katalysierten Bedingungen (Houben-Weyl, Band VI/3 205, 221).

Um unter Verwendung von verzweigten Aldehyden oder Ketonen befriedigende Ausbeuten zu erhalten, wird empfohlen, mehrere Mole des Alkohols, z.B. drei Mole Alkohol oder mehr bezogen auf die Carbonylverbindung einzusetzen. Auf diese Weise erhält man mit dem sterisch anspruchslosen aliphatischen Keton Aceton 73-75 % Ausbeute, während das stärker verzweigte Pikolin lediglich eine Ausbeute von 41 % liefert (siehe Houben-Weyl, Band VI/3, 222 und 223).

Diese verhältnismäßig niedrigen Ausbeuten als auch der Einsatz von mehreren Moläquivalenten Alkohol bei der Ketaldarstellung ist aus ökonomischen als auch ökologischen Gründen sehr nachteilig. Die unvollständig ablaufenden Umsetzungen erfordern nämlich einerseits großtechnisch nur schwer realisierbare aufwendige destillative Trennungen des Reaktionsgemischs wegen ähnlicher Siedepunkte der enthaltenen Komponenten während andererseits die nach der Destillation anfallenden größeren Rückstände entsorgt werden müssen (siehe Houben-Weyl, Band VI/3, 210).

Wie aus der nachfolgenden Tabelle 1 ersichtlich ist, muß man beispielsweise zur Aufarbeitung der ablaufenden Synthese von 2,2-Dimethoxy-3-methylbutan nach dem Claisen-Verfahren eine destillative Auftrennung von mindestens fünf Substanzen vornehmen, die alle in einem Temperaturintervall von maximal 90 °C sieden:

Tabelle 1

| Verbindung | Kp. (°C) | Produkt/Edukt |
|---|---|---|
| Ameisensäuremethylester | 36 | Produkt |
| Methanol | 64-65 | Edukt/Produkt |
| 3-Methylbutanon-2 | 94-96 | Edukt |
| Orthoameisensäuretrimethylester | 100-102 | Edukt |
| 2,2-Dimethoxy-3-methyl-butan | 122-126 | Produkt (gewünscht) |

Die (destillative) Reinigung der nach diesem literaturbekannten Verfahren hergestellten Ketale ist in der Regel unumgänglich, wenn man in nachfolgenden Stufen der Verarbeitung der Ketale befriedigende Ausbeuten erzielen will.

Ein weiterer Nachteil der destillativen Reinigung besteht darin, daß man die als Katalysator zugesetzten

Säuren mit Basen neutralisieren muß, da andernfalls unter den sauren Bedingungen bei der Destillation die Ketale unter Bildung von nicht erwünschtem Enolether zersetzt werden. Dies bedeutet eine zusätzliche Abwasserbelastung durch Neutralisationssalze.

Eine weitere Möglichkeit, die nicht vollständig in die Ketale überführten Carbonylverbindungen aus dem Reaktionsmedium abzutrennen, besteht darin, die Carbonylverbindungen chemisch auszuwaschen (siehe Houben-Weyl, Band VI/3, 210). Dies führt aber beim großtechnisch angewandten Verfahren aus ökologischer und ökonomischer Sicht zu untragbaren Abwasserbelastungen.

Erschwerend für die Verbesserung der Ausbeute kommt hinzu, daß Alkohole und Carbonylverbindungen sehr reaktive Substrate darstellen, die viele Nebenreaktionen unerwünschter Art eingehen können.

Die obengenannten Nachteile der bekannten Verfahren lassen sich überraschenderweise durch das im folgenden beschriebenen erfindungsgemäße Verfahren vermeiden.

Gegenstand der vorliegenden Erfindung ist ein Verfahren zur Herstellung von Verbindungen der Formel (I)

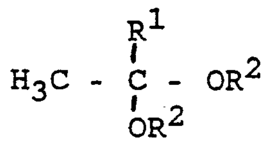

(I)

worin

$R^1$ Wasserstoff, Alkyl, Alkenyl oder Alkinyl, vorzugsweise $C_1$-$C_6$-Alkyl, insbesondere Propyl, und

$R^2$ Alkyl, vorzugsweise $C_1$-$C_2$-Alkyl,

bedeuten, dadurch gekennzeichnet, daß man eine Verbindung der Formel (II)

$R^1$ - CO - $CH_3$ (II)

worin

$R^1$ die obengenannte Bedeutung besitzt, mit katalytischen Mengen der Verbindung der Formel (III) und mindestens äquimolaren Mengen der Verbindung der Formel (IV)

$R^2OH$ (III)

$HC(OR^2)_3$ (IV)

unter sauren Bedingungen und Abdestillieren des entstehenden Ameisensäureesters der Formel $HCOOR^2$ zu Verbindungen der Formel (I) umsetzt.

Alkyl bedeutet dabei einen geradkettigen, verzweigten oder cyclischen Kohlenwasserstoffrest, Alkenyl einen entsprechenden Kohlenwasserstoffrest mit ein oder mehreren Doppelbindungen, Alkinyl einen entsprechenden Kohlenwasserstoffrest mit ein oder mehreren Dreifachbindungen, der auch noch Doppelbindungen enthalten kann und Halogenalkyl entsprechende Alkylreste, die ein-oder mehrfach durch Halogenatome, wie Fluor, Chlor, Brom oder Jod, insbesondere Fluor oder Chlor, substituiert sind.

Geeignete Alkylreste für $R^1$ sind beispielsweise Methyl, Ethyl, n-Propyl, i-Propyl, n-, i-, sek- und tert.-Butyl sowie die Pentyl- und Hexylisomeren, Cyclopentyl und Cyclohexyl. Unter den aufgeführten Resten $R^1$ ist besonders i-Propyl bevorzugt.

Geeignete Alkinylreste für $R^1$ sind Ethinyl, Prop-1-inyl und But-1-inyl.

Geeignete Reste für $R^2$ sind beispielsweise die obengenannten Alkylreste; vorzugsweise bedeutet $R^2$ jeweils unabhängig vom anderen Rest $R^2$, Methyl oder Ethyl, insbesondere nur Methyl.

Das erfindungsgemäße Verfahren kann beispielsweise so durchgeführt werden, daß man das Keton (II) mit saurem Katalysator und katalytischen Mengen Alkohol (III) vorlegt, den Orthoameisensäureester (IV) möglichst kontinuierlich zudosiert und den größten Teil des entstehenden Ameisensäureesters laufend abdestilliert. Der Alkohol (III) und/oder der saure Katalysator kann bzw. können auch ganz oder teilweise, zusammen oder parallel mit dem Orthoameisensäureester (IV) in das Reaktionsgemisch dosiert werden.

Überraschenderweise wird der Alkohol (III) unter diesen Reaktionsbedingungen nicht mit abdestilliert, sondern reagiert rasch quantitativ mit dem Keton der Formel (II).

Die Reaktionstemperaturen liegen bei der zur Destillation des Ameisensäureesters erforderlichen Temperatur, wobei durch Überhitzung und Siedepunktserhöhung die Temperatur der Reaktionsmischung höher, meist bis zu 30 °C höher liegen kann als der Siedepunkt des entsprechenden Ameisensäureesters. Beispielsweise durch Wahl des Ameisensäureesters sowie des Drucks kann die Temperatur noch variiert werden. Vorzugsweise liegt die Temperatur bei 20 bis 100 °C, insbesondere 40 bis 80 °C.

Die sauren Reaktionsbedingungen können beispielsweise durch Zusatz von anorganischen oder organischen Säuren oder Puffersystemen, vorzugsweise in katalytischen Mengen, erreicht werden.

Als katalytisch eingesetzte Säuren kommen die in der Fachliteratur genannten Säuren wie z.B. $H_2SO_4$,

para-Toluolsulfonsäure, Essigsäure, saure Ionenaustauscher und Ammoniumchlorid in Frage, die praktisch wasserfrei sind und unter den Reaktionsbedingungen nicht oder nur in geringen Mengen abdestilliert werden. Die erforderliche und optimale Menge an Säure läßt sich im Einzelfall leicht durch faktorielle Versuchsplanung ermitteln.

Das erfindungsgemäße Verfahren wird mit katalytischen Mengen an Alkohol (III) von zweckmäßig höchstens 0,1 Mol pro Mol Keton (II), vorzugsweise von 0,01 bis 0,08, insbesondere von 0,03 bis 0,06 Mol Alkohol (III) pro Mol Keton (II) durchgeführt. Die Reaktionskomponenten werden vorzugsweise praktisch wasserfrei eingesetzt.

Der Orthoameisensäureester (IV) wird vorzugsweise in einer Menge von 1 bis 1,1 Mol, insbesondere 1,04 bis 1,07 Mol pro Mol Keton (II) eingesetzt.

Nach dem erfindungsgemäßen Verfahren lassen sich die Ketale der Formel (I) in nahezu quantitativen Ausbeuten von in der Regel über 98 % d. Th. und in so hohen Reinheiten darstellen, daß eine destillative Reinigung der Ketale nicht mehr notwendig wird, sondern die Rohprodukte direkt in den nachfolgenden chemischen Synthesen eingesetzt werden können. Der eingesetzte Orthoameisensäureester dient als Reaktant und generiert den Ameisensäureester, der nach dem erfindungsgemäßen Verfahren kontinuierlich aus dem Reaktionsmedium durch Destillation entfernt wird.

Bei dieser Verfahrensweise benötigt man im Gegensatz zu den literaturbekannten Verfahren nur noch katalytische Mengen an Alkohol, um selbst bei sperrigen und sterisch anspruchsvollen Ketonen das entsprechende Ketal in praktisch quantitativen Ausbeuten darzustellen.

Überraschenderweise reagieren die Ketone der Formel (II) mit den Alkoholen so rasch, daß selbst unter den destillativen Bedingungen nach dem erfindungsgemäßen Verfahren, unter denen die Ameisensäureester aus dem Reaktionsmedium kontinuierlich abdestilliert werden, kein Alkohol mit abdestilliert wird.

Das erfindungsgemäße Verfahren ist durch die nachfolgenden Beispiele erläutert.

**Beispiel 1**

In einem 2 l-Rührkolben mit einer 20 cm-Vigreuxkolonne als Destillationsaufsatz werden 438 g 3-Methylbutanon-2, 9,0 g para-Toluolsulfonsäure und 8,0 g Methanol vorgelegt und, bei 40 °C beginnend, mit insgesamt 571 g Orthoameisensäuretrimethylester innerhalb von 2 Stunden versetzt, während gleichzeitig der entstehende Ameisensäuremethylester abdestilliert wird. Die Reaktionslösung wird für 2 Stunden bei 60 bis 70 °C nachgerührt, wobei die restlichen Mengen Ameisensäuremethylester und das eingesetzte Methanol abdestilliert werden. Es werden insgesamt 324 g Leichtsieder als Destillat abdestilliert. Die Reaktionstemperatur liegt im Bereich von 40 °C bis 70 °C. Der Siedepunkt des abdestillierten Destillats beträgt 36 °C (= Sdp. Ameisensäuremethylester) bis ca. 60 °C (= Sdp. Methanol). Es werden 672 g Destillationsrückstand erhalten, der 98,4 Gew.-% (98,4 % d. Th.) an 2,2-Dimethoxy-3-methylbutan enthält

**Beispiel 2**

In einem 2 l-Rührkolben mit 20 cm-Vigreuxkolonne als Destillationsaufsatz werden 876 g 3-Methylbutanon-2, 60 g stark saurer Ionenaustauscher (Dowex 50 WX4) und 16,0 g Methanol vorgelegt und, bei 40 °C beginnend, mit insgesamt 1142 g Orthoameisensäuretrimethylester innerhalb von 2 Stunden versetzt, währenddessen der entstehende Ameisensäuremethylester abdestilliert wird.

Die Reaktionslösung wird für 2 Stunden bei 60 °C bis 70 °C nachgerührt, wobei die restlichen Mengen Ameisensäuremethylester und das eingesetzte Methanol abdestilliert werden. Es werden insgesamt 62,2 g Leichtsieder als Destillat abdestilliert. Die Reaktionstemperatur liegt im Bereich zwischen 40 und 70 °C. Der Siedepunkt des abdestillierten Destillats beträgt 36 °C (Sdp. Ameisensäuremethylester) bis ca. 60 °C (Sdp. Methanol). Es werden 1348 g Destillationsrückstand erhalten. Von diesem Destillationsrückstand werden die 60 g des stark sauren Ionenaustauschers abfiltriert, die in einem nachfolgenden Ansatz direkt wieder eingesetzt werden können. Der Destillationsrückstand enthält 98 Gew.-% an 2,2-Dimethoxy-3-methylbutan, was einer Ausbeute von 98,4 % d. Th. entspricht.

**Beispiel 3**

In einem 4 l-Rührkolben mit 20 cm-Vigreuxkolonne als Destillationsaufsatz werden 876 g 3-Methylbutanon-2, 18 g Ammoniumchlorid und 16 g Methanol vorgelegt und, bei 40 °C beginnend, mit 1142

g Orthoameisensäuretrimethylester innerhalb von 3 Stunden versetzt, währenddessen der entstehende Ameisensäuremethylester weitgehend kontinuierlich abdestilliert wird. Die Reaktionslösung wird für 2 Stunden bei 60 bis 70 °C nachgerührt und dabei die restlichen Mengen an Ameisensäuremethylester und das eingesetzte Methanol abdestilliert.

Es werden insgesamt 640 g Leichtsieder als Destillat abdestilliert. Die Reaktionstemperatur liegt im Bereich von 40 bis 70 °C. Der Siedepunkt des abdestillierten Destillats beträgt 36 °C bis ca. 60 °C. Es werden 1353 g Destillationsrückstand erhalten, der 97,2 Gew.-% (97,9 % d. Th.) an 2,2-Dimethoxy-3-methylbutan enthält.

**Vergleichsbeispiel** (analog Houben-Weyl, Band VI/3, S. 223)

137,6 g 3-Methylbutanon-2, 260,8 g Orthoameisensäuretrimethylester, 153,6 g Methanol und 8 g Ammoniumchlorid werden bei Zimmertemperatur eine Woche stehengelassen. Danach wird vom Ammoniumchlorid abfiltriert, mit Äther nachgewaschen und zweimal mit einer 0 °C kalten Calciumchloridlösung zur Entfernung des Alkohols gewaschen.

Die Reaktionslösung wird über Calciumchlorid getrocknet und anschließend der Äther und Ameisensäuremethylester abdestilliert. Nach Zugabe von gepulvertem, wasserfreiem Natriumcarbonat wird das Produkt bei 122 bis 126 °C destilliert. Man erhält insgesamt 129,6 g Produkt, das 98,9 Gew.-% 2,2-Dimethoxy-3-methylbutan (60,6 % d. Th.) enthält.

**Ansprüche**

1. Verfahren zur Herstellung der Verbindungen der Formel (I),

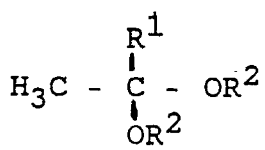

(I)

worin
$R^1$ Wasserstoff, Alkyl, Alkenyl oder Alkinyl und
$R^2$ Alkyl,
bedeuten, dadurch gekennzeichnet, daß man eine Verbindung der Formel (II),
$R^1 - CO - CH_3$ (II)
worin
$R^1$ die obengenannte Bedeutung besitzt, mit katalytischen Mengen der Verbindung der Formel (III) und mindestens äquimolaren Mengen der Verbindung der Formel (IV)
$R^2OH$ (III)
$HC(OR^2)_3$ (IV)
unter sauren Bedingungen und Abdestillieren des entstehenden Ameisensäureesters der Formel $HCOOR^2$ zu Verbindungen der Formel (I) umsetzt.

2. Verfahren nach Anspruch 1, dadurch gekennzeichnet, daß
$R^1$ $C_1$-$C_6$-Alkyl und
$R^2$ $C_1$-$C_2$-Alkyl bedeuten.

3. Verfahren nach Anspruch 1 oder 2, dadurch gekennzeichnet, daß
$R^1$ i-Propyl und
$R^2$ Ethyl oder Methyl bedeuten.

4. Verfahren nach einem oder mehreren der Ansprüche 1 bis 3, dadurch gekennzeichnet, daß der Alkohol der Formel (III) in einer Menge von höchstens 0,1 Mol pro Mol Keton der Formel (II) eingesetzt wird.

5. Verfahren nach Anspruch 4, dadurch gekennzeichnet, daß 0,01 bis 0,08 Mol Alkohol (III) pro Mol Keton (II) eingesetzt wird.

6. Verfahren nach einem oder mehreren der Ansprüche 1 bis 5, dadurch gekennzeichnet, daß die sauren Bedingungen durch Zusatz von katalytischen Mengen einer Säure aus der Gruppe $H_2SO_4$, p-Toluolsulfonsäure, Essigsäure, saurer Ionenaustauscher und Ammoniumchlorid eingestellt werden.

7. Verfahren nach einem oder mehreren der Ansprüche 1 bis 6, dadurch gekennzeichnet, daß 1,0 bis 1,1

Mol Orthoameisensäureester (IV) pro Mol Keton der Formel (II) eingesetzt wird.

8. Verfahren nach einem oder mehreren der Ansprüche 1 bis 7, dadurch gekennzeichnet, daß man die Reaktion bei Temperaturen von 20 °C bis 100 °C durchführt.

9. Verfahren gemäß Anspruch 8, dadurch gekennzeichnet, daß man die Reaktion bei Temperaturen von 40 bis 80 °C durchführt.

Europäisches Patentamt

# EUROPÄISCHER RECHERCHENBERICHT

Nummer der Anmeldung

**EP 90 11 4123**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,A | HOUBEN-WEYL: "METHODEN DER ORGANISCHEN CHEMIE" 4. Auflage, Band VI/3, Seiten 222-225, 1965, Stuttgart, DE<br>* Seite 222, Absatz 2 * | 1 | C 07 C 41/56<br>C 07 C 43/303 |
| A | EP-A-0 046 282 (J.T. BAKER CHEMICAL CO.)<br>* Seiten 19,20; "preparation A" * | 1 | |
| A | US-A-3 803 244 (A.A. SCHLEPPNIK)<br>* Spalte 3, Zeilen 17-21 * | 1 | |

RECHERCHIERTE SACHGEBIETE (Int. Cl.5)

C 07 C 41/00
C 07 C 43/00

Der vorliegende Recherchenbericht wurde für alle Patentansprüche erstellt

| Recherchenort | Abschlußdatum der Recherche | Prüfer |
|---|---|---|
| Berlin | 23 November 90 | PROBERT C.L. |

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze

E: älteres Patentdokument, das jedoch erst am oder nach dem Anmeldedatum veröffentlicht worden ist
D: in der Anmeldung angeführtes Dokument
L: aus anderen Gründen angeführtes Dokument

&: Mitglied der gleichen Patentfamilie, übereinstimmendes Dokument